# EUROPEAN PATENT APPLICATION

(11) **EP 0 655 477 A1**
(43) Date of publication of application: **31.05.1995**
(21) Application number: 94117741.2
(22) Date of filing: 10.11.1994
(51) Int. Cl.: C08J 7/12, G01N 33/545

(54) **Method for obtaining articles with improved properties for immunological assays**

(30) Priority: 26.11.1993 IT MI932506
(71) Applicant: BIOMAT S.n.c., I-38068 Rovereto, Trento (IT)
(72) Inventor: Pettenati, Maurizia, I-38068 Rovereto, Trento (IT); Marmieri, Gabriele, I-38068 Rovereto, Trento (IT)
(74) Representative: Luksch, Giorgio, Dr.-Ing.

(57) **Abstract**

A cold plasma method for modifying the surface chemical structure of conventional polystyrene articles for biological assays, the operating conditions of which are such that the surface structure of the polystyrene article has a surface oxygen content of between 0.5 and 8% and preferably between 2.5 and 3%.

## Description

The invention relates to a method for forming articles for immunological assays.

A large number of current chemical-clinical evaluations are based on so-called solid-phase immunological assays. As is known to the expert of the art, solid-phase immunological assays utilize articles, generally constructed of polymeric material and more specifically of polystyrene, which perform the double function of containing the fluids and reagents for the analysis, and of acting as an interface on which the reagents for originating the reaction on which the analysis is based are initially fixed. These articles can be of different forms, the most common of which are test tubes, spheres, microwells formed in a single plate and strips of microwells fixed onto a frame. A typical immunological analysis scheme comprises fixing (technically known as "adsorbing") for example an antigen onto microwells. The sample to be analyzed, which can contain the antibody for this antigen, is then added. In this case the antigen and antibody form a complex, the presence of which is detected by adding suitable reagents which generally develop a coloration used as the analytical signal. If the presence of a given antigen is to be detected, a suitable antibody has to be adsorbed onto the surface of the article used for the solid-phase immunological assay.

From this brief description it is apparent that the effectiveness and sensitivity of a detection system for the immunological assay require the combination of certain important aspects. Firstly the antigen-antibody biochemical system must be optimized, as must all the reagents used for detecting the analyzed substance. Again, the surface of the solid phase, consisting of the article used for the immunological assay, must have high capacity for adsorbing biological molecules. This characteristic results in precision and reproducibility of the measurement, even when working with very low concentrations of the analyzed substance. A further desirable characteristic of the article is that it maintains the bond with the biological molecules even in the presence of surfactants, which are normally used in immunological assays during washing to eliminate those weakly and unstably adsorbed biological molecules which could provide spurious signals during detection. In addition, as the analytical signal is generally detected by spectrophotometric measurements, the solid substrate must have a high degree of transparency. Basically the fundamental characteristics of materials used for the articles utilized in immunological assays as the solid phase are their capacity to stably bind in high quantity the biological molecules present in the analytical solutions and their optical quality. For these reasons, the material chosen to form articles for solid-phase immunological assays is polystyrene, which provides high transparency and a good level of interaction with biological molecules.

In evolving and improving the methods used in solid-phase immunological assays it has however proved apparent that the characteristics of polystyrene, although good, have not been completely satisfactory in certain aspects. In particular, improvements have been sought in its capacity to adsorb biological molecules present in very low concentrations and in the resistance of adsorbed molecules to removal by washing with surfactants.

As described for example by J. D. Andrade in the second volume of the text "Surface and Interfacial Aspects of Biomedical Polymers", published by Plenum Press, New York, 1985, the capacity of a synthetic phase to adsorb biological molecules and the relative force of interaction are controlled by the chemical composition of the most outer (surface) layers of the material. For this reason a series of treatments has been devised for modifying the chemical composition of the surface of articles for immunological assays, in order to improve their interaction with biological molecules. One of the treatments used, as declared by S. E. Rasmussen in the article published in 1990 in Annals of Biological Clinic, volume 48, page 647, is to treat polystyrene articles with gamma rays. Articles for immunological assays treated in this manner, known to the expert as "high binding capacity" articles, are effectively distinguished by better biological molecule adsorption than untreated polystyrene. Although satisfactory, treatment with gamma rays has certain negative aspects. Firstly irradiation with gamma rays involves the need for a radioactive source, usually the isotope Co⁶⁰. The presence of this source implies a definite risk to man and the environment and requires a series of precautions to minimize the possible effects of the radioactive material. These precautions, which consist for example of building work of suitable characteristics and the use of authorized personnel, require the availability of suitable sites and spaces, and are extremely costly. A further negative aspect of gamma ray treatment is the formation, on the treated articles, of a brown-yellow coloration, a side effect of the interaction between gamma rays and polystyrene. As is known to the expert, the presence of this coloration is immediately apparent on visually comparing a conventional article for immunological assays with one of high binding capacity, as heretofore defined. In addition to its negative appearance, this coloration results in a reduction in the transparency of the material. As already stated, transparency is one of the most important characteristics required of materials for immunological assay articles.

An alternative method to irradiation with gamma rays is chemical treatment. In this case the article or rather the material with which it is formed is immersed for a certain time in a solution of liquids which have to be highly aggressive because of the chemical nature of polymeric materials. For example a polystyrene surface can be modified by immersion in 96% sulphuric acid. Although effective, this treatment cannot be used for producing articles on an industrial scale because of the danger of the chemical compound, the possibility of pollution and problems in disposing the effluent from the treatment.

A non-polluting surface modification method is treatment by the technology known as "cold plasma". This treatment is also used to modify the surface of polystyrene articles used in cell cultures, as described for example in an article by B. Ratner, A. Chilcoti and G. P. Lopez published in the book "Plasma Deposition, Treatment and Etching of Polymers", published by R. D'Agostino, Academic Press, New York, 1990, in which the resultant modified surface comprises more than 12% of oxygen. Unfortunately the resultant surface characteristics of cell culture articles do not favour adsorption of the biological molecules commonly present in immunological assay formulations, and in this respect the properties of such articles are inferior to those of analogous articles formed of untreated polystyrene.

From the aforegoing description it is apparent that there is a need for a surface modification method which improves the characteristics of conventional articles without prejudicing other positive characteristics and without environmental and pollution risk or danger.

The present applicant has now surprisingly found that such a method exists and consists of treating conventional immunological assay articles with cold plasma, under specific operating conditions specified in the accompanying claims. This treatment results in immunological assay articles in which interaction with the molecules of the biological phase is similar to or better than that of "high binding capacity" articles as heretofore defined, without presenting coloration or loss of transparency. The treatment does not require the use of radioactive sources or aggressive or dangerous chemical compounds, and does not give rise to environmental or pollution risks.

In its widest aspect, the invention consists of the treatment of conventional articles for immunological assays with cold plasma under specific operating conditions, and of the products obtained in this manner. In particular, the invention consists of a process for treating conventional articles for immunological assays using cold plasma technology, this process being controlled in such a manner as to impart a well defined chemical composition to the article surface. More specifically, the cold plasma treatment conditions must be controlled in such a manner as to introduce onto the article surface an oxygen quantity of between 0.5 and 8%, and preferably between 2 and 5%. This modification of the surface chemical composition is achieved by controlling the cold plasma treatment conditions, as described hereinafter.

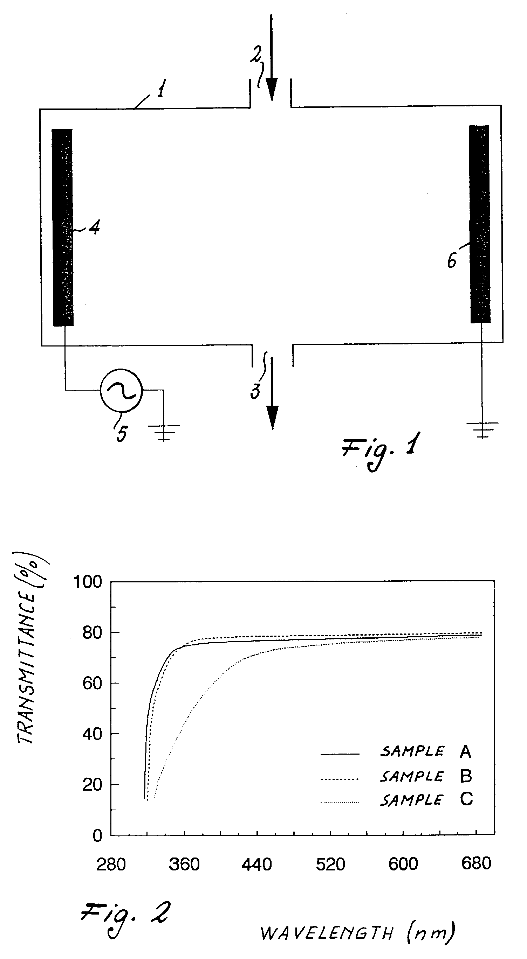

The cold plasma (hereinafter known simply as "plasma") technique and its application to the surface modification of polymeric materials are described, for example, in the text "Polymer Interface and Adhesion" by S. Wu, published by Marcel Dekker in 1982. A simplified scheme of a plasma treatment reactor is shown in Figure 1. It consists of a reaction chamber 1, generally of glass, steel or aluminium construction, provided with a process gas or vapour access system 2 and an outlet connected to the pumping system 3. This pumping system enables vacuum to be applied to the interior of the reaction chamber 1. Electrodes are inserted into this latter. One electrode, 4, is connected to an energy source, usually a radiofrequency or microwave energy source 5. The other electrode, 6, is connected to earth. The plasma treatment, as taught by S. Wu in the cited book, is effected by feeding the required gas or vapour into the previously evacuated reaction chamber. The gaseous phase is brought to the plasma state by providing it with a suitable power by means of the energy source.

According to a particularly favourable aspect of the present invention, the conventional articles for immunological assays are placed in the plasma treatment reactor. At this point vacuum is created, which according to the invention is between 0.000001 and 1 Torr and preferably between 0.0001 and 0.1 Torr. The process gas or vapour or the process gas or vapour mixture is then introduced until the system pressure reaches a value between 0.001 and 100 Torr and preferably between 0.005 and 10 Torr. The type of gas or vapour, or gas or vapour mixture, is non-limiting. Any element or compound or mixture can be used which under the aforedefined process conditions is present in gaseous form, provided said element or compound or mixture is able to introduce onto the article surface the aforedefined quantity of oxygen atoms. Indicative but non-limiting examples of the elements or compounds which can be used are O₂, H₂, N₂, N₂O, Ar, He, H₂O, CH₃OH, CH₃COCH₃ or their mixtures. The operating conditions are controlled by the operator in accordance with prior determination in such a manner as to obtain the desired chemical composition on the article surface. The use of milder conditions, in particular a lesser power and treatment time, with chemically more active gases such as O₂, and more energetic conditions in the case of less reactive gases or vapours such as N₂, Ar or CH₃OH, fully fall within the spirit of the invention.
Non-limiting examples of conditions for the treatment according to the present invention are the use of a mixture composed of 99% Ar and 1% O₂, at an operating pressure of 0.235 Torr, a power of 66 Watt and a treatment time of 12 seconds. This treatment enables an oxygen quantity of 3% to be introduced onto the surface of conventional articles for immunological assays, providing superior biological molecule adsorption characteristics. Moreover, the emissions from this treatment consist solely and completely of unreacted O₂ and Ar, and therefore present no environmental problem.

The importance of the invention will be apparent to the expert of the art. The invention enables immunological assay articles of high binding capacity to be obtained by a non-polluting treatment without effluents or environmental risks. The articles obtained by the described treatment present no coloration or reduction in transparency.

Some illustrative examples are given hereinafter, without representing a limitation on the scope of the invention.

### EXAMPLE 1

A conventional article for immunological assays (Mikrotestplatte FB96, manufactured by Ratiolab, Germany), in the form of a 96 microwell plate, is subjected to plasma treatment defined by the following conditions: mixture Ar 99%/O₂ 1%, pressure 0.235 Torr, power 66 W, treatment time 12 seconds. The treatment is conducted in a plasma treatment reactor PS 0350 manufactured by the firm Plasma Science (California, USA). The treated article is defined as sample A.

A second conventional article for immunological assays (Mikrotestplatte FB96, manufactured by Ratiolab, Germany), in the form of a 96 microwell plate, is subjected to plasma treatment defined by the following conditions: Ar 100%, pressure 0.5 Torr, power 34 W, treatment time 60 seconds. The treatment is conducted in the aforedescribed reactor. The treated article is defined as sample A1.

A third conventional article for immunological assays (Mikrotestplatte FB96, manufactured by Ratiolab, Germany), in the form of a 96 microwell plate, is subjected to plasma treatment defined by the following conditions: mixture N₂ 90%/O₂ 10%, pressure 0.1 Torr, power 40 W, treatment time 10 seconds. The treatment is conducted in the aforedescribed reactor. The treated article is defined as sample A2.

The treated articles are subjected to a biological evaluation test with the following other articles, also in the form of 96 microwell plates:
Sample B: conventional article for immunological assays (Mikrotestplatte FB96, manufactured by Ratiolab, Germany)
Sample C: high binding capacity article for immunological assays (Nunc Maxisorb by Nunc, Denmark)
Sample D: treated article for cell culture growth (Corning TM).

The biological evaluation test is structured in the following manner: human immunoglobulin G (IgG) proteins are introduced into the wells in a concentration of 0.15 aeg/I. New-born calf serum is then added, followed by various washes. Anti-GG (AIgG) is then added in a concentration of 1/1000, 1/2500, 1/5000 and 1/10000. The analytical signal, indicating the IgG quantity adsorbed and hence the binding capacity of the article, is obtained by adding para-nitrophenylphosphate in diethanolamine. In the presence of the said proteins this compound forms a typical coloration, the intensity of which is proportional to the protein quantity adsorbed. The intensity of the analytical signal is measured by a microplate reader manufactured by Molecular Devices (USA). A wavelength of 405 nm is used for the reading. The optical density measured at this wavelength is proportional to the protein quantity adsorbed.
The following results are obtained:

| Sample | AIgG concentration | Optical density |
|---|---|---|
| A | 1/1000 | 100 |
| A | 1/2500 | 80 |
| A | 1/5000 | 30 |
| A | 1/10000 | 8 |
| A1 | 1/1000 | 98 |
| A1 | 1/2500 | 81 |
| A1 | 1/5000 | 34 |
| A1 | 1/10000 | 6 |
| A2 | 1/1000 | 103 |
| A2 | 1/2500 | 88 |
| A2 | 1/5000 | 34 |
| A2 | 1/10000 | 10 |
| B | 1/1000 | 90 |
| B | 1/2500 | 40 |
| B | 1/5000 | 5 |
| B | 1/10000 | 0 |
| C | 1/1000 | 103 |
| C | 1/2500 | 85 |
| C | 1/5000 | 32 |
| C | 1/10000 | 8 |
| D | 1/1000 | 68 |
| D | 1/2500 | 19 |
| D | 1/5000 | 2 |
| D | 1/10000 | 0 |

The results show that the described treatments considerably increase the binding capacity of conventional articles, rendering them similar to high binding capacity materials without however the drawbacks thereof. At the same time it is apparent that treated articles for cell culture growth are not able to provide the properties required for conducting immunological assays.

### EXAMPLE 2

The conventional sample B of Example 1 is subjected to the following plasma treatment in the reactor used for Example 1: gas N₂, pressure 0.500 Torr, power 90 W, treatment time 20 seconds. This sample is defined as sample E. The biological evaluation test gives the following results:

| Sample | AIgG concentration | Optical density |
|---|---|---|
| E | 1/1000 | 97 |
| E | 1/2500 | 65 |
| E | 1/5000 | 27 |
| E | 1/10000 | 8 |

### EXAMPLE 3

The samples of the preceding examples are subjected to surface analysis by X-ray photoelectronic spectroscopy, the instrument used being a Perkin Elmer PHI 5500. The analysis gives the following results:

| Sample | Surface composition (atomic %) | |
|---|---|---|
| | O | C |
| A | 2.8 | 97.2 |
| A1 | 2.0 | 98.0 |
| A2 | 4.9 | 95.1 |
| B | 0.2 | 99.8 |
| C | 3.1 | 96.9 |
| D | 12.4 | 87.6 |
| E | 3.0 | 97.0 |

### EXAMPLE 4

The transparency of samples A, B and C of Example 1 is evaluated by measuring the transmission spectrum in the visible and near ultraviolet range, using a UV-visible Unicam 8710 spectrophotometer.

The measured spectra, shown in Figure 2, demonstrate the transparency loss of the high binding capacity sample, whereas there is no significant difference between the spectra of the conventional article and that treated by plasma.

## Claims

1. A method for obtaining polystyrene articles with improved properties for immunological assays, characterised by consisting of treating such articles in a cold plasma reactor so as to introduce onto their surface an oxygen quantity of between 0.5% and 8% and preferably between 2.5% and 3%.

2. A method as claimed in claim 1, wherein after placing the article or articles in the cold plasma reactor, a vacuum of between 0.000001 Torr and 1 Torr and preferably between 0.0001 Torr and 0.1 Torr is created therein.

3. A method as claimed in claim 1, characterised in that a gaseous oxidizing agent is introduced into the cold plasma reactor until a system pressure of between 0.001 Torr and 100 Torr and preferably between 0.005 Torr and 10 Torr is obtained.

4. A method as claimed in claim 3, wherein the oxidizing agent is a mixture of Ar and O₂, the O₂ percentage being between 0.5 and 10%.

5. A method as claimed in claim 3, wherein the oxidizing agent consists of CH₃OH vapour.

6. A method as claimed in claim 3, wherein the oxidizing agent is air.

7. A method as claimed in claim 1, wherein the power used in the reactor is between 5 and 80 W.
